# EUROPEAN PATENT SPECIFICATION

(11) **EP 4 143 574 B1**
(45) Date of publication and mention of the grant of the patent: **11.12.2024**
(21) Application number: 21717087.7
(22) Date of filing: 09.04.2021
(51) Int. Cl.: G01N 33/569

(54) **IN VITRO METHOD FOR DIAGNOSING SUBJECTS INFECTED WITH MYCOBACTERIUM SPECIES**
IN-VITRO-VERFAHREN ZUR DIAGNOSE VON MIT MYCOBACTERIUM-SPEZIES INFIZIERTEN PERSONEN
PROCÉDÉ IN VITRO POUR LE DIAGNOSTIC DES SUJETS INFECTÉS PAR L'ESPÈCE MYCOBACTERIUM

(30) Priority: 13.04.2020 EP 20382291
(43) Date of publication of application: 08.03.2023
(73) Proprietor: NEIKER - Instituto Vasco de Investigación y Desarrollo Agrario, S.A., 48160 Derio, Bizkaia (ES); SERIDA, Servicio Regional de Investigación y Desarrollo Agroalimentario, 33300 Villaviciosa, Asturias (ES)
(72) Inventor: ALONSO FERNÁNDEZ-PACHECO, Marta, 48160 Derio, Bizkaia (ES); CASAIS GOYOS, Rosa, 33394 Gijón, Asturias (ES); JUSTE JORDÁN, Ramón, 48160 Derio, Bizkaia (ES); BLANCO VÁZQUEZ, Cristina, 33394 Gijón, Asturias (ES); CANIVE RUIZ, María, 48160 Derio, Bizkaia (ES); IGLESIAS BESTEIRO, Natalia, 33394 Gijón, Asturias (ES)
(74) Representative: Igartua, Ismael
(86) International application number: PCT/EP2021/059316
(87) International publication number: WO 2021/209339

(56) References cited:
- CN-A- 110 172 508
- MARTA ALONSO-HEARN ET AL: "RNA-Seq analysis of ileocecal valve and peripheral blood from Holstein cattle infected with Mycobacterium avium subsp. paratuberculosis revealed dysregulation of the CXCL8/IL8 signaling pathway", SCIENTIFIC REPORTS, vol. 9, no. 1, 16 October 2019 (2019-10-16), XP055726968, DOI: 10.1038/s41598-019-51328-0
- MOREIRA-TEIXEIRA LÚCIA ET AL: "Mouse transcriptome reveals potential signatures of protection and pathogenesis in human tuberculosis", NATURE IMMUNOLOGY, NATURE PUBLISHING GROUP US, NEW YORK, vol. 21, no. 4, 16 March 2020 (2020-03-16), pages 464 - 476, XP037075245, ISSN: 1529-2908, [retrieved on 20200316], DOI: 10.1038/S41590-020-0610-Z
- CATHERINE W. M. ONG ET AL: "Neutrophil-Derived MMP-8 Drives AMPK-Dependent Matrix Destruction in Human Pulmonary Tuberculosis", PLOS PATHOGENS, vol. 11, no. 5, 21 May 2015 (2015-05-21), pages e1004917, XP055726982, DOI: 10.1371/journal.ppat.1004917
- NATHELLAP KUMAR ET AL: "Elevated levels of matrix metalloproteinases reflect severity and extent of disease in tuberculosis-diabetes co-morbidity and are predominantly reversed following standard anti-tuberculosis or metformin treatment", BMC INFECTIOUS DISEASES, BIOMED CENTRAL LTD, LONDON, UK, vol. 18, no. 1, 25 July 2018 (2018-07-25), pages 1 - 10, XP021258950, DOI: 10.1186/S12879-018-3246-Y
- SHIVANI SINGH ET AL: "Antimycobacterial Drugs Modulate Immunopathogenic Matrix Metalloproteinases in a Cellular Model of Pulmonary Tuberculosis", ANTIMICROBIAL AGENTS AND CHEMOTHERAPY, vol. 58, no. 8, 2 June 2014 (2014-06-02), US, pages 4657 - 4665, XP055726983, ISSN: 0066-4804, DOI: 10.1128/AAC.02141-13

## Description

### FIELD OF THE INVENTION

The present invention refers to the medical or veterinary field. Particularly, the present invention is directed to an *in vitro* method for the diagnosis of subjects infected with *Mycobacterium* species, particularly subjects infected with *Mycobacterium avium subsp. paratuberculosis* (MAP) which is the causative agent of paratuberculosis (PTB).

### STATE OF THE ART

MAP is a pathogenic bacterium which pertains to the genus *Mycobacterium.* MAP causes Johne's disease or PTB, which mainly affects ruminants such as cattle, and it has been associated with some human diseases such as Crohn's disease, ulcerative colitis or colorectal cancer [Pierce ES et al., 2018. Could Mycobacterium avium subspecies paratuberculosis cause Crohn's disease, ulcerative colitis... and colorectal cancer? Infect Agent Cancer. 2018 Jan 4;13:1. doi: 10.1186/s13027-017-0172-3. eCollection 2018*] [*Garvey M et al., 2018. Mycobacterium avium subspecies paratuberculosis: A possible causative agent in human morbidity and risk to public health safety. Open Vet J. 2018;8(2):172-181. doi: 10.4314/ovj.v8i2.10. Epub 2018 May 19*].* Moreover, MAP has been associated with an increasingly long list of inflammatory/autoimmune diseases: sarcoidosis, Blau syndrome, Hashimoto's thyroiditis, autoimmune diabetes (T1D), multiple sclerosis (MS), rheumatoid arthritis, lupus and Parkinson's disease [Dow CT et al., 2020. Proposing BCG Vaccination for Mycobacterium avium ss. paratuberculosis (MAP) Associated Autoimmune Diseases. Microorganisms. 2020 Feb 5; 8(2). PMID: 32033287 PMCID: PMC7074941].

Transmission of MAP primarily occurs by the fecal-oral route through the ingestion of MAP contaminated feces, colostrum, or milk. Infection usually occurs within the first months of live of the animal but remains sub-clinical for an average of 2-5 years. After being ingested, MAP crosses the intestinal mucosa where it is phagocytosed by sub-epithelial macrophages establishing a chronic infection. MAP is able to survive and proliferate within phagosomes by inhibiting apoptosis and phagosome acidification and by preventing presentation of antigens to the immune system. As the infection progresses, the lesions in the intestine and mesenteric lymph nodes become more severe. Rather than focalized, the granulomatous infiltrate becomes diffuse disrupting the mucosal structure and function and affecting jejunum and ileum. According to their location and extension, the histological lesions present in the intestinal tissues of infected animals are classified into focal, multifocal, and diffuse categories [González J et al., 2005. Histopathological classification of lesions associated with natural paratuberculosis infection in cattle. J Comp Pathol. 2005 Aug-Oct; 133(2-3):184-96. PMID: 16045917 DOI: 10.1016/j.jcpa.2005.04.007]. The focal lesions consist of small granulomas, mainly located in the jejunal and ileal lymph nodes. The multifocal lesions consist of well-demarcated granulomas in the intestinal lymphoid tissue and also in the intestinal lamina propia. The diffuse lesions were characterized by severe granulomatous enteritis and lymphadenitis. According to the inflammatory cell type present in the infiltrate and the amount of acid-fast bacilli (AFB), diffuse lesions were subdivided into diffuse lymphoplasmacytic or paucibacillary, intermediate and diffuse histiocytic or multibacillary lesions [Balseiro et al., 2018. Chronic regional intestinal inflammatory disease: A trans-species slow infection? Comp. Immunol., Microbiol., Infect. Dis. 2018. 62:88-100. DOI: https://doi.org/j.cimid.2018.12.001]. In the diffuse lymphoplasmacytic lesion type the cellular infiltrate is composed mainly of lymphocytes that extends through broad areas, contains rare AFBs and is clearly visible to the naked eye. The diffuse histiocytic lesion type consists of large number epithelioid cells, lymphocytes, macrophages, and numerous Langhans giant cells with huge amounts of AFBs, that affect large areas of the intestine and appears as a thickening of the intestinal wall readily visible upon opening the intestine. The diffuse intermediate type can also be seen under the naked eye affecting broad areas of the intestine as a thickening of the mucosa microscopically composed of lymphocytes, macrophages and scant Langhans giant cells with small numbers of AFB.

PTB is responsible for significant economic losses in dairy herds worldwide due to decreased milk production, increased management costs and premature culling from clinical disease. Several studies have demonstrated that more than 50 % of the dairy cattle herds are positive for MAP antibodies in USA and in Europe and, therefore, bovine PTB can be considered endemic in these areas. Commercial inactivated vaccines against bovine PTB are very successful in reducing MAP presence in feces and tissues and in increasing both milk production and cattle productive life in infected farms when compared with unvaccinated farms. However, PTB vaccination with heat-killed inactivated vaccines is not allowed in most European countries due to its interference with *Mycobacterium bovis* detection tests. Therefore, PTB control is currently based on testing and culling plus preventing MAP transmission to susceptible animals using appropriate hygienic-sanitary strategies. However, the efficiency of the control programs based on the "test and cull" policy is strongly conditioned by the sensitivity of the tests used to identify early infections. Presently, fecal culture is considered "the gold standard" test for the ante-mortem diagnosis of MAP infection. However, individual fecal culture is expensive and time consuming and only detects advanced infections due to the relatively late onset of fecal shedding during the natural course of MAP infection. In fact, the sensitivity of the fecal culture is 70 % in animals with PTB-associated clinical signs, but only 23-29 % in animals with sub-clinical infection, which may shed MAP intermittently and in lower numbers in feces and milk contaminating the environment and infecting other animals.

Early-stage diagnostics such as the *IFN-γ* release assay (IGRA) detects whether a T-cell mediated immune response has been elicited in response to mycobacterial antigens. The IGRA method uses a sandwich ELISA to detect the cellular response to MAP by measuring the difference in *IFN-γ* signals for whole blood samples activated by specific antigen (MAP extract) and non-specific antigen (M. *phlei* extract) (ID Vet). The IGRA only reflects MAP exposure, and thus cannot discriminate between individuals with controlled infection from those with sub-clinical disease.

The IDEXX ELISA, which detects specific antibodies produced against MAP, is nowadays widely used for the diagnosis of PTB. However, the IDEXX ELISA is associated with a high rate of false negative results (low sensitivity) *[*Vazquez et al., 2014. Latent infections are the most frequent form of paratuberculosis in slaughtered Friesian cattle. Spanish Journal of Agricultural Research 2014 12(4): 1049-1060. http://dx.doi.org/10.5424/sjar/2014124-5978]. This means that a high percentage of subjects, those latently infected, still test negative. False negative results are a concern because they hamper the implementation of control or eradication measures.

So, the detection of MAP infections, mainly sub-clinical infections before the bacterium is shed and transmitted to herd mates and possibly to humans, is still an unmet need. In other words, novel tools with high sensitivity and specificity are needed to detect MAP-infections, mainly at early stages of the infection and during the long choric phase that precedes the clinical stage of the disease.

The present invention is focused on solving the above cited problems and it is herein provided a new strategy for the diagnosis of subjects infected with MAP, which offers a high sensitivity and specificity, also at early stages or during the long chronic stage of the MAP infection.

### DESCRIPTION OF THE INVENTION

### Brief description of the invention

Such as it has been explained above, the present invention is directed to an *in vitro* method for the diagnosis of subjects infected with *Mycobacterium* species, particularly subjects infected with MAP which is the causative agent of PTB.

Five of the many biomarkers identified by RNA-Seq were selected for the development of novel ELISAs for diagnosis purposes, according to their high expression levels in animals with focal or diffuse histological lesions with respect to the control animals without lesions.

**Table 1** shows differential expression of host biomarker genes selected after RNA-Seq analysis of peripheral blood of Holstein cattle with focal and diffuse histological lesions versus control animals without detected lesions. The mean Log2 fold change is a measurement of differential gene expression between two compared groups.

**Table 1**

| **Biomarker ID** | **Gene ID** | **Focal vs. Control** | | **Diffuse vs. Control** | |
|---|---|---|---|---|---|
| | | Mean Log2 fold change | p-value | Mean log2 fold change | p-value |
| MMP8 | XLOC_010350 | 1.89 | 0.00035 | | |
| ABCA13 | XLOC_033714 | 3.74 | 0.00005 | | |
| FAM84A | XLOC_005071 | | | 2.15 | 0.00005 |
| SPARC | XLOC_040915 | | | 2.21 | 0.00065 |
| DES | XLOC_019473 | | | 3.75 | 0.00030 |

| | | | | | |
|---|---|---|---|---|---|
| *MMP8, bovine matrix metallopeptidase 8; ABCA13, bovine ATP binding cassette subfamily A member 13; FAM84A, bovine family with sequence similarity 84-member A; SPARC, bovine secreted protein acidic and cystein rich; DES. bovine desmin.* | | | | | |

Genes encoding for bovine proteins MMP8 (Matrix metallopeptidase 8) and ABCA13 (ATP binding cassette subfamily A member 13) showed significantly higher expression in animals with focal lesions, while genes encoding for bovine proteins FAM84A (family with sequence similarity 84-member A), SPARC (secreted protein acidic and cystein rich) and DES (desmin) were up-regulated in animals showing diffuse lesions. Biomarker selection was based on high levels of expression, cellular location (extracellular proteins, present in sera, were the preferred choice for detection by ELISA) and commercial availability of ELISA kits for its specific detection.

The diagnostic potential of five ELISAs designed to detect MMP8, ABCA13, FAM84A, SPARC and DES was studied using serum samples from infected cows with distinct histological lesions in gut tissues representing various stages of the disease. The gold standard or reference technique used to classify the animals included in this study was the type of histological lesions present in their intestinal tissues. Significantly, such as it can be seen in the results provided below, it was not possible to validate the five biomarker-based ELISAs for the diagnosis of PTB. In fact, only three out of the five biomarker-based ELISAs were able to discriminate animals with focal lesions, two out of the five biomarker-based ELISAs biomarkers discriminated animals with multifocal lesions, two out of the five biomarker-based ELISAs discriminated animals with diffuse lesions and three out of the five biomarker-based ELISAs were able to discriminate all the infected animals from the control group. Indeed, the ELISAs for the detection of FAM84A and DES did not discriminate any group of infected animals (their AUC was always below 0.7).

Of note, the ELISAs for the detection of proteins ABCA13 and/or SPARC and /or MMP8 showed a high performance, specifically to discriminate the group of animals with focal lesions and all-lesions group (which comprises all the animals with focal, multifocal and diffuse histological lesions). Such as it is shown in the results provided below, the ELISA for the detection of the ABCA13 showed the most accurate diagnostic performance for both the detection of animals with focal lesions (AUC value of 0.866, p<0.001) and for the overall detection of animals with any type of histological lesions (AUC value of 0.825, p<0.001). Thus, the ELISA for the detection of ABCA13 is an accurate method for detection of animals with focal lesions and for overall detection of animals with any type of histological lesion.

Consequently, ABCA13 is herein presented as a reliable alternative for the methods currently used for the diagnosis of PTB. In fact, IDEXX ELISA is nowadays widely used for the diagnosis of PTB although this method is associated with a high rate of false negative results (low sensitivity). This means that a high percentage of infected subjects still test negative for the disease due to the low sensitivity of IDEXX ELISA. This problem is solved by the present invention because, such as it is herein explained, the ABCA13 showed a high sensitivity and specificity.

So, the first embodiment of the present invention refers to an in *vitro* method for diagnosing subjects infected with *Mycobacterium* species which comprises: a) Measuring the concentration of the protein ABCA13 in a biological sample consisting of serum or plasma obtained from the subject, and b) wherein if an increase of the concentration of the protein ABCA13 is identified in the step a) with respect to the concentration of the protein ABCA13 measured in uninfected control subjects, it is an indication that the subject is infected with *Mycobacterium* species.

In a preferred embodiment, the present invention refers to an *in vitro* method for diagnosing subjects infected with MAP which comprises: a) Measuring the concentration of the protein ABCA13 in a biological sample consisting of serum or plasma obtained from the subject, and b) wherein if an increase of the concentration of the protein ABCA13 is identified in the step a) with respect to the concentration of the protein ABCA13 measured in uninfected control subjects, it is an indication that the subject is infected with MAP.

In a preferred embodiment, the present invention refers to an *in vitro* method for the diagnosis of PTB in a subject which comprises: a) Measuring the concentration of the protein ABCA13 in a biological sample consisting of serum or plasma obtained from the subject, and b) wherein if an increase of the concentration of the protein ABCA13 is identified in the step a) with respect to the concentration of the protein ABCA13 measured in uninfected control subjects, it is an indication that the subject is suffering from PTB.

In a preferred embodiment, the above-described *in vitro* methods comprise: a) Measuring the concentration of the proteins [ABCA13 and SPARC] in a biological sample consisting of serum or plasma obtained from the subject, and b) wherein if an increase of the concentration of the proteins [ABCA13 and SPARC] is identified in the step a) with respect to the concentration of the proteins [ABCA13 and SPARC] measured in uninfected control subjects, it is an indication that the subject is suffering from PTB.

In a preferred embodiment, the above-described *in vitro* methods comprise: a) Measuring the concentration of the proteins [ABCA13 and SPARC and MMP8] in a biological sample consisting of serum or plasma obtained from the subject, and b) wherein if an increase of the concentration of the proteins [ABCA13 and SPARC and MMP8] is identified in the step a) with respect to the concentration of the proteins [ABCA13 and SPARC and MMP8] measured in uninfected control subjects, it is an indication that the subject is suffering from PTB.

In a preferred embodiment, the above-described *in vitro* methods comprise: a) Measuring the concentration of the proteins, in a biological sample consisting of serum or plasma obtained from the subject, wherein if the concentration obtained for any of the previously cited proteins is above (≥) the established cut-off value, this is indicative that the subject is suffering from PTB.

In a preferred embodiment, the above-described *in vitro* methods comprise the diagnosis of latent or patent PTB, preferably latent PTB.

In a preferred embodiment, the subject is a mammal selected from the group consisting of: cattle, goat, sheep, horse, pig, deer, rabbit, wild boar, bison, llama, alpaca, opossum, badger, elephant or human being; preferably a farm-animal selected from the group consisting of: cow, goat, sheep, horse, pig, deer, llama or alpaca.

In a preferred embodiment, the above cited method is an immunoassay, preferably enzyme-linked immunosorbent assay (ELISA).

The second embodiment of the present invention refers to the *in vitro* use of the protein ABCA13, of the proteins [ABCA13 and SPARC], or of the proteins [ABCA13 and SPARC and MMP8], in a biological sample consisting of serum or plasma, for diagnosing subjects infected with *Mycobacterium* species.

In a preferred embodiment, the proteins are used for diagnosing subjects infected with MAP.

In a preferred embodiment, the proteins are used for the diagnosis of PTB.

In a preferred embodiment, the proteins are used for the diagnosis of latent or patent PTB, preferably latent PTB.

The third embodiment of the present invention refers to the use of a kit which comprises tools or reagents for determining the concentration the protein ABCA13 in a biological sample consisting of serum or plasma, for diagnosing subjects infected with *Mycobacterium* species.

In a preferred embodiment, the present invention refers to the use of a kit which comprises tools or reagents for determining the concentration of the proteins [ABCA13 and SPARC] or the proteins [ABCA13 and SPARC and MMP8] in a biological sample consisting of serum or plasma, for diagnosing subjects infected with *Mycobacterium* species.

The disclosure further refers to a method for treating subjects infected with *Mycobacterium species,* particularly with MAP, by applying a therapeutically effective dose or amount of a composition which brings about a positive therapeutic response in a subject infected with the bacteria. Significantly, this method comprises a first step wherein the subject is diagnosed by using any of the methods described above.

The disclosure further refers to a method for guaranteeing that a batch of milk comes from females free of MAP infection and therefore that the product is apt for human consumption without any significant risk of causing any of the above-mentioned human diseases.

For the purpose of the present invention the following terms are defined:
- The sentence "concentration of the proteins measured in uninfected control subjects", refer to a "reference value" of the concentration of the proteins. If an "increase" of the concentration of the proteins is determined with respect to said "reference value" (i.e. the concentration is significantly higher than the reference value), this is an indication that the subject is infected with the bacteria.
- A "reference value" can be a threshold value or a cut-off value. Typically, a "threshold value" or "cut-off value" can be determined experimentally, empirically, or theoretically. A "cut-off value" can also be arbitrarily selected based upon the existing experimental and/or clinical conditions, as would be recognized by a person of ordinary skilled in the art. The "cut-off value" has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The ROC curve is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between positive and negative results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis/prognosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Any existing software or systems in the art may be used for the drawing of the ROC curve.
- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of'. Thus, the phrase "consisting of" indicates that the listed elements are required or mandatory, and that no other elements may be present.
- By "therapeutically effective dose or amount" of a composition for treating PTB is intended an amount that, when administered, brings about a positive therapeutic response in a subject suffering from PTB. The exact amount required will vary from subject to subject, depending on the species, age, and general condition of the subject, the severity of the condition being treated, mode of administration, etc. An appropriate "effective" amount in any individual case may be determined by one of ordinary skill in the art using routine experimentation, based upon the information provided herein.
- "Latent" and "patent" refer to the two possible kinds of infections caused by MAP. "Latent" PTB refers to the presence of focal lesions with low or moderate frequency of microbiological or immunological evidence of infection. A "latent" infection would give rise to a chronic or subclinical manifestation of the disease wherein the clinical signs are still not perceptible. In contrast, "patent" PTB refers to the presence of multifocal or diffuse inflammatory lesions with a high frequency of microbiological or immunological evidence of infection. A "patent" infection would generally give rise to a clinical manifestation of the disease, wherein the clinical signs are perceptible.

### Description of the figures

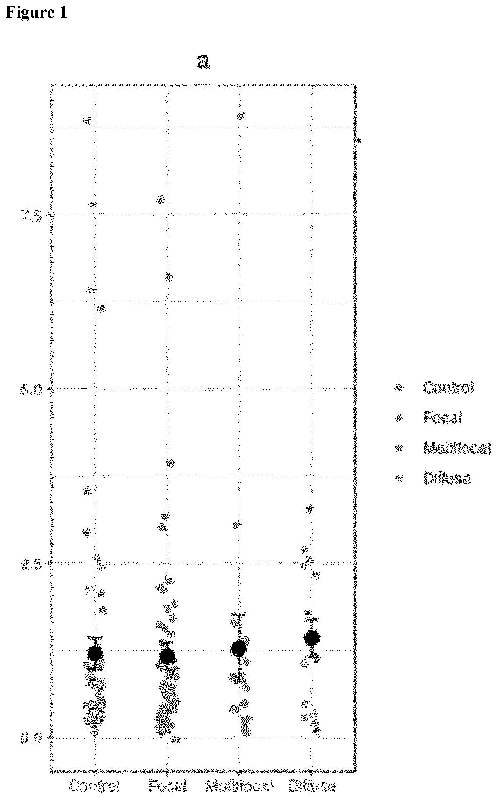

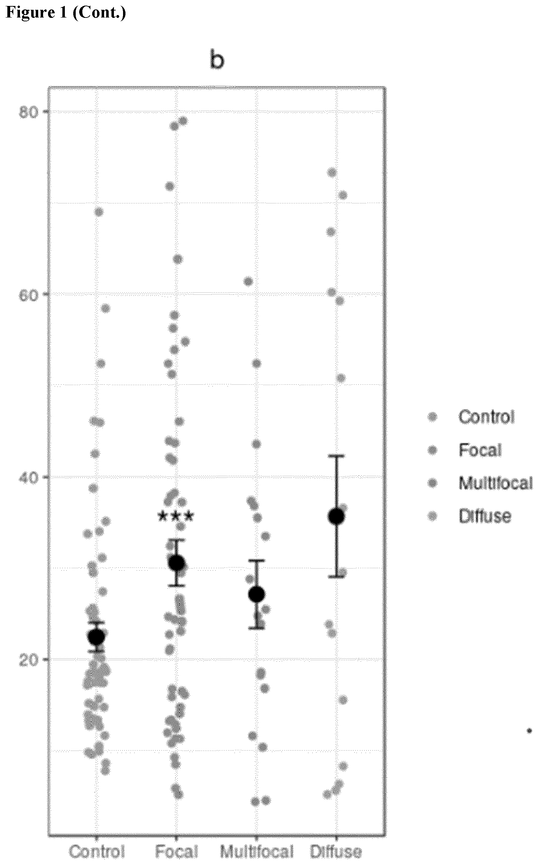

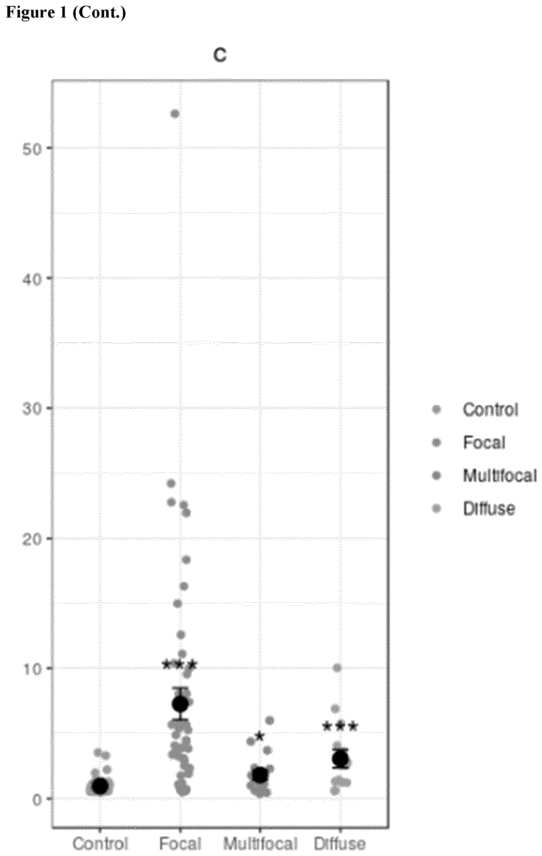

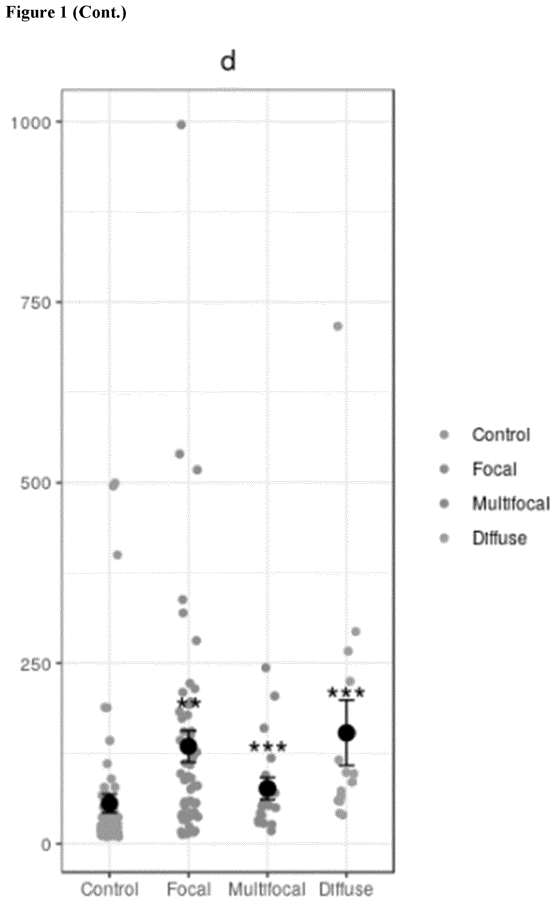

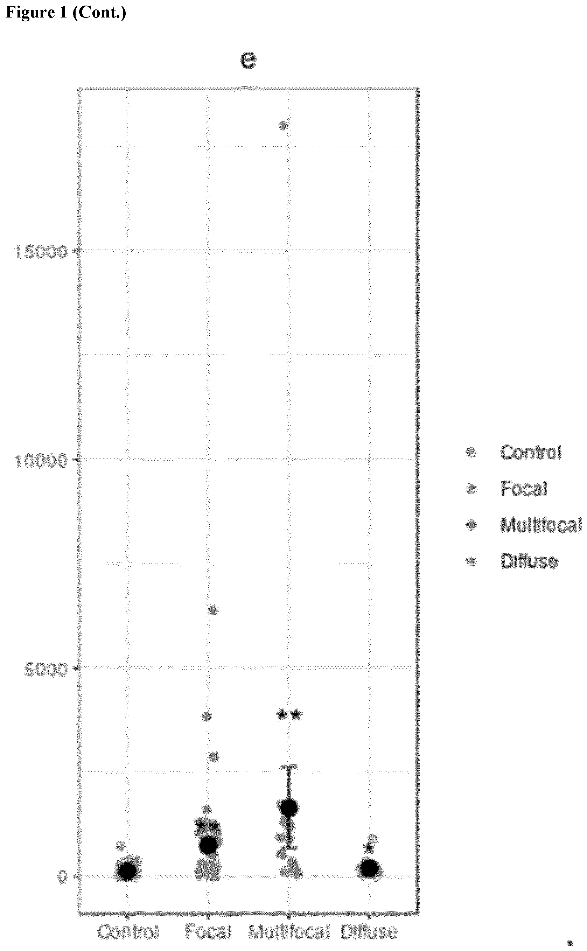

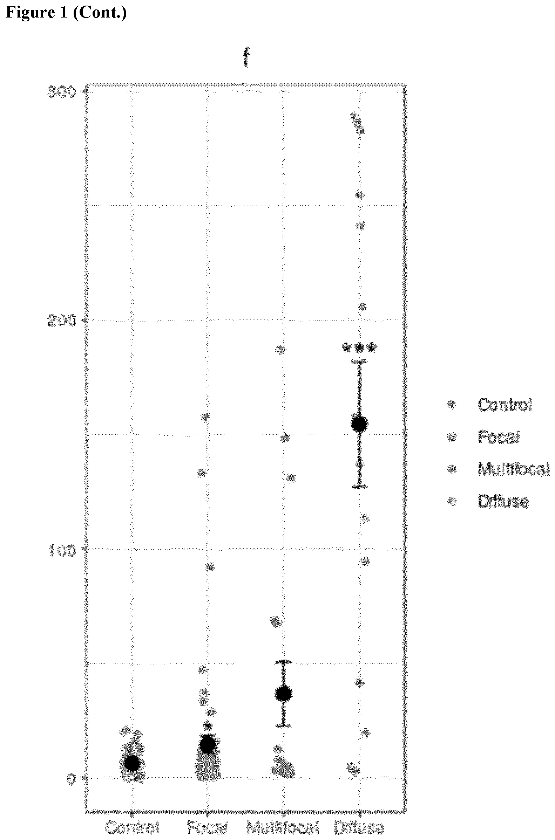

**Figure 1**. Biomarker concentration levels in serum of Holstein Friesian cattle showing different types of histological lesions in the intestinal tissues focal (n=55), multifocal (n=17), and diffuse (n=15). Control animals (n=61) were animals from a PTB-free farm. The number of animals with focal, multifocal, diffuse or with any type of lesions analysed for ABCA13 was 53, 16, 14 and 83, respectively. Biomarkers were detected and quantified in bovine serum by specific ELISAs supplied by MyBioSource, San Diego, CA, USA. a) FAM84A, bovine family with sequence similarity 84-member A; b) DES, bovine desmin; c) ABCA13, bovine ATP binding cassette subfamily A member 13; d) MMP8, bovine matrix metallopeptidase 8; e) SPARC, bovine secreted protein acidic and cystein rich; f) IDEXX ELISA. The data are represented as scatter plots with each dot representing a single animal. The mean of each histological group is represented by a gross black point and the standard deviation by a vertical line. The asterisks indicate if the differences between each histological group and the control are or not significant (*, p<0.05; **, p>0.01; ***, p<0.001).

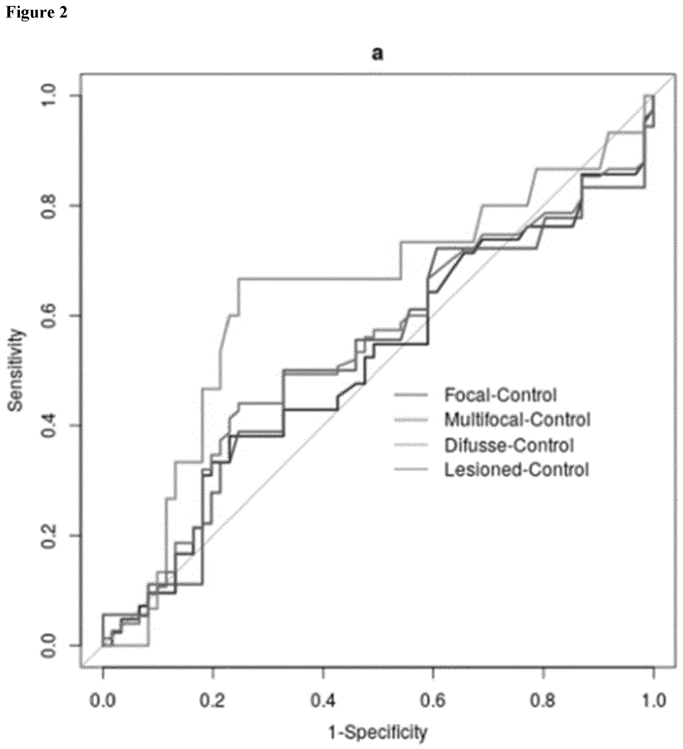

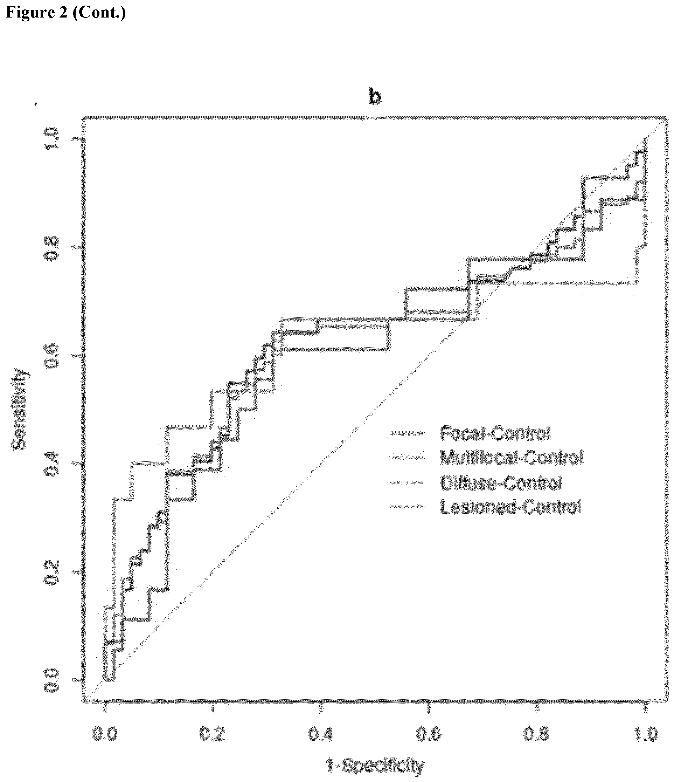

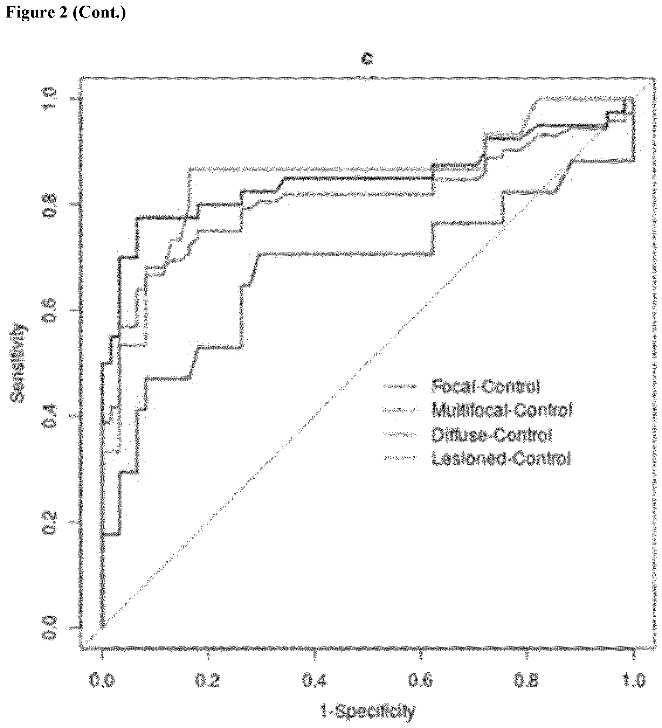

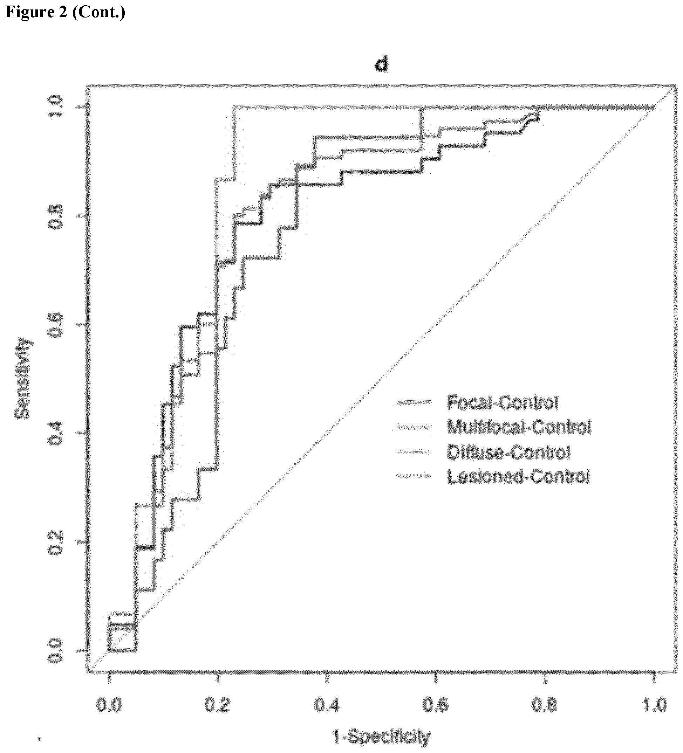

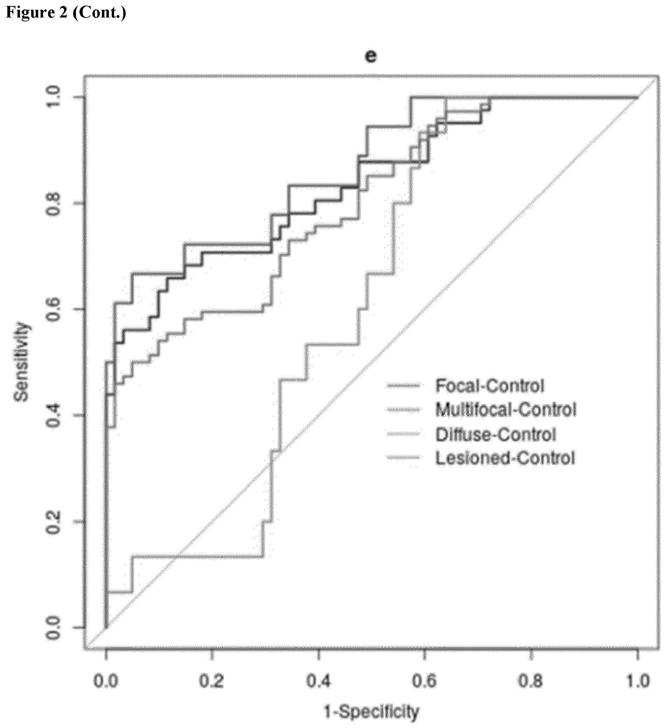

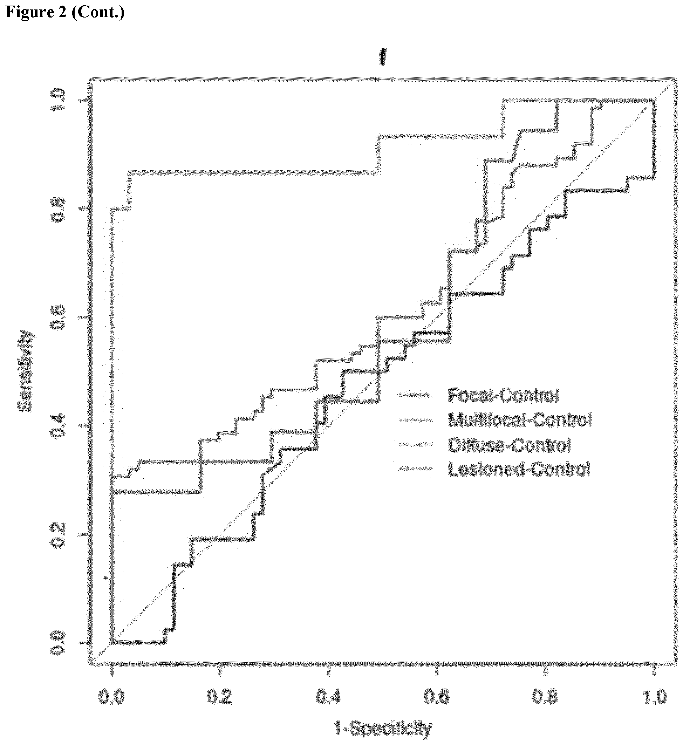

**Figure 2****.** Receiver operator characteristics curves (ROC curves) of selected biomarkers in Holstein Friesian cows with focal (n=55), multifocal (n=17) or diffuse (n=15) histological lesions in their intestinal tissues versus control animals from a PTB-free farm (n=61). The number of animals with focal, multifocal, diffuse or with any type of lesions analysed for ABCA13 was 53, 16, 14 and 83, respectively, **a)** FAM84A, bovine family with sequence similarity 84-member A; **b)** DES, bovine desmin, **c)** ABCA13, bovine ATP binding cassette subfamily A member 13; **d)** MMP8, bovine matrix metallopeptidase 8; **e)** SPARC, bovine secreted protein acidic and cystein rich; **f)** IDEXX serum ELISA.

### Detailed description of the invention

The following examples disclose a preferred way of carrying out the invention, without the intention of limiting its scope of protection.

### Example 1. Methodology.

### Example 1.1 Animals and ethical considerations

Two groups of animals were included in this field study: **Slaughtered group)** Ninety-three Holstein Friesian cows (ranging from 0.81 to 12.66 years of age) came from 26 farms located in the Principality of Asturias (Northwest of Spain). In Asturias 25% of the herds and 1.9% of the animals were positive by serum ELISA in 2017 (data taken from the records of the Regional Government). Specifically, fifty-five animals came from a dairy farm with a mean herd size of 105 cows (2016-2019) and a mean prevalence of PTB of 6.30% in the sampling period, based on serum ELISA test (IDEXX laboratories, Hoofddorp, The Netherlands). Another thirty-four animals were chosen randomly from cows slaughtered in the local abattoir (coming from 24 different farms) and the remaining four cows came from SERIDA's Friesian cow farm (3% mean prevalence of PTB in the period 2018-2019). The PTB infection status of these 93 animals at the time of slaughter was determined by histopathology, serum ELISA test, and bacteriological culture and specific real-time PCR of tissues and feces. Information related to the presence of PTB-associated clinical signs such as gradual weight loss, diarrhea and decreased milk production was obtained from the farmers when possible; **and PTB-free group)** Sixty-one animals (ranging from 0.50 to 10.08 years of age) from a PTB-free farm in Asturias were used as the control group for the study. The PTB-free status of this control farm was verified yearly by IDEXX serum ELISA, the absence of clinical cases in the period 2016-2020 as well as by the absence of positive fecal bacteriological culture and PCR results in 2019.

Experimental procedures were approved by the SERIDA Animal Ethics Committee and authorised by the Regional Consejeria de Agroganadería y Recursos Autóctonos del Principado de Asturias. Spain (authorization codes PROAE 29/2015 and PROAE 66/2019). All the procedures were carried out in accordance with the Directive 2012/63/EU of the European Parliament.

### Example 1.2. Tissues and fecal real-time polymerase chain reaction (PCR)

Isolation of genomic DNA from tissues and feces was performed using the MagMax Total Nucleic Acid Isolation kit according to the manufacturer's instructions (TermoFisher Scientifc, Lissieu, France). For detection of MAP DNA, the LSI VetMax Triplex real-time PCR was used according to the manufacturer's instructions (TermoFisher Scientifc, Lissieu, France). The kit enables real-time PCR detection of *Map* IS900 and F57 genes in DNA extracted from feces, liquid cultures, and tissues or colonies. Real-time PCR amplifications were performed using the MX3000P Real-Time PCR detection system (Stratagene, San Diego, USA) system with the following conditions: 1 cycle at 50 °C for 2 min, 1 cycle of 95 °C for 10 min, 45 cycles of denaturation at 95 °C for 15 s, and annealing/extension at 60 °C for 60 s.

### Example 1.3. Bacteriological culture of tissues and feces

For bacteriological culture, a pool (2 gr) of ileocecal lymph nodes, distal jejunal lymph node, ileocecal valve (ICV), and distal jejunum were decontaminated with 38 mL of hexa-decyl pyridinium chloride at a final concentration of 0.75% (Sigma, St. Louis, MO) and homogenized in a stomacher blender. After 30 min of incubation at room temperature, 15 mL of the suspension was transferred to a new tube and incubated overnight for decontamination and sedimentation. Approximately, 200 µl of the suspension was taken from the layer near the sediment and inoculated into two slants of Herrolds egg yolk medium (HEYM; Becton Dickinson, Sparks, MD) and into two slants of Lowenstein-Jensen medium (LJ; Difco, Detroit, MI), both supplemented with 2mg/L of Mycobactin J (ID.vet Innovative Diagnostics, Grabels, France). At the time of slaughter, feces were taken from the rectum of each animal, maintained at 4°C and processed within 48 h after arrival at the laboratory. The fecal samples (2 g each) were decontaminated, blended in a stomacher, and cultured in HEYM and LJ, as previously described for tissue culture.

### Example 1.4. Histological classification of animals

As mentioned above animals included in this study were classified according to the type of histological lesions present in their intestinal tissues. Tissue sections of distal jejunum, ICV and jejunum and ICV lymph nodes were collected from the 93 slaughtered cows, fixed in 10% neutral buffered formalin, sliced and embedded in paraffin wax using standard procedures. Afterwards, 4 µm sections were assessed by haematoxylin-eosin (HE) and Ziehl-Neelsen (ZN) staining for specific acid-fast bacteria detection. Slices were observed using an Olympus BH-2 light microscope and photographed using an Olympus DP-12 digital camera. The stained sections were examined by light microscopy for detection and classification of pathological lesions and for the presence of acid-fast bacteria (AFB).

### Example 1.5 Enzyme-Linked Immunosorbent Assays (ELISA) for detection of the selected biomarkers

Peripheral blood was collected from the tail vein of all the cows included in the study using BD Vacutainer Z serum clot activator Plastic tubes (Vacuette. Kremsmunster. Austria). After clotting, serum was separated by centrifugation for 20 min at 2,500g at room temperature and stored at -20 °C until use. The concentrations of the selected biomarkers in the serum of each animal were measured using commercially available ELISAs according to the manufacturers' instructions. Quantitative sandwich ELISA kits Bovine Matrix Metalloproteinase 8 (MMP8) (Detection range 3.12-100 ng/mL); Bovine Protein FAM84A ELISA kit (Detection range 62.5-2000 pg/mL), Bovine SPARC ELISA kit (Detection range 0.78-50 ng/mL), and competitive Bovine ATP-binding cassette subfamily A member 13 (ABCA13) ELISA kit (Detection range 0-5000 pg/mL) and Bovine Desmin (DES) ELISA kit (Detection range 0-25 ng/mL) were used for specific detection of MMP8, FAM84A, ABCA13, SPARC and DES, respectively (MyBioSource, San Diego, CA. USA).

The assay procedure for the specific detection of biomarker ABCA13 is described in more details as follows:
1. Add 100 µL of serum samples and the standards to the appropriate well. Add 100 µL of PBS (pH 7.0-7.2) to the blank control well.
2. Add 50 µL of conjugate to each well (NOT to the blank control well). Mix well. Cover the plate and incubate for 1 hour at 37°C.
3. Wash the plate five times with diluted 1X wash solution (350-400 µL /well/wash) using an automatic washer. It is recommended that the washer be set for a soaking time of 10 seconds and shaking time of 5 seconds between each wash.
4. Add 50 µL substrate A and 50 µL substrate B to each well including blank control well. Subsequently, cover and incubate for 15 minutes at 37°C (avoid sunlight, if after 15 min the color is not dark please prolong the incubation time, but the longest time is 30min).
5. Add 50 µL of Stop Solution to each well including blank control well. Mix well.
6. Determine the Optical Density (O.D.) at 450 nm using a microplate reader immediately.

A standard curve was used to determine the concentration of biomarkers in the serum samples (average OD of each standard was plotted on the vertical axis against the concentration on the horizontal axis and the best fit drawn to generate a regression curve). Standards and samples were tested in duplicate. The mean value of the blank control was subtracted from mean raw OD values before result interpretation. For optimization various dilutions of the serum were tested (for instance: undiluted, 1:2, 1:4 and 1:8) and the dilution which showed a larger number of samples with measurement values included within the range of the standard curve was considered optimal. In most cases the optimal dilution was 1:2, however, samples with high concentrations of a specific biomarker had to be assayed at higher dilutions.

### Example 1.6 Statistical analysis.

The AUC (area under the curve) and optimal cut-off value for each ELISA was determined by Receiver operator characteristic (ROC) curve analysis. The optimal cut off values for sensitivity and specificity were based on maximum Youden Index (J=Se+Sp-1).

The discriminatory power of each biomarker-based ELISA to differentiate between the different histological groups and the control group was determined according to [Muller, M.P., Tomlinson, G., Marrie, T.J., Tang, P.., McGeer, A., Low, D.E., Detsky, A.S., Gold, W.L. (2005). Can routine laboratory tests discriminate between severe acute respiratory syndrome and other causes of community-acquired pneumonia? Clin Infect Dis. 40(8):1079-86. https://doi.org/10.1086/428577][ParkHE., Park, H T., Jung YH., Yoo, HS. (2017). Establishment a real-time reverse transcription PCR based on host biomarkers for the detection of the sub-clinical cases of Mycobacterium avium subsp. Paratuberculosis. PLoS One. 25;12(5):e0178336. doi: 10.1371/journal.pone.0178336] as follows: AUC scores≥0.9 were considered to have excellent discriminatory power; 0.8≤AUC <0.9 good discriminatory power; 0.7≤AUC <0.8 fair discriminatory power; and AUC <0.7, poor discriminatory power (Muller et al., 2005). Higher AUC scores were considered as showing better discriminatory powers.

Multivariate binary logistic regression models (Caret package of R) were used to assess the diagnostic capacity of the simultaneous use of several biomarkers, providing the AUC, and values like sensitivity and specificity for the different biomarker combinations. Comparison of ROC curves to test the statistical significance of the difference between the areas under ROC curves (derived from the same cases) was performed with the method of DeLong et al. (1988) [DeLong ER, DeLong DM, Clarke-Pearson DL (1988): Comparing the areas under two or more correlated receiver operating characteristic curves: a nonparametric approach. Biometrics 44:837-845*]* for the biomarkers with fair, good and excellent AUC values (AUC≥0.7). Statistical significance of differences in quantitative variables (for instance: age) between two histological groups were studied using the t Student test (normality) or Wilcoxon test (not normality).

All the data was analyzed using the pROC, OptimalCutpoints and Caret [Max Kuhn (2020). caret: Classification and Regression Training. R package version 6.0-85. https://CRAN.R-project.org/package=caret] packages of R Statistical environment version 3.6.0 [R Core Team (2018). R: A language and environment for statistical computing. R Foundation for Statistical Computing. Vienna. Austria. URL https://www.R-project.org/], with confidence intervals stated at 95%.

### Example 2. Results.

### Example 2.1 Histological, immunological and microbiological assessment of MAP infection status

The histological, immunological and microbiological characteristics of all the animals included in this study (slaughtered animals n=93 and control animals from a PTB-free farm n=61) are summarized in **Table 2.**

Thus, **Table 2** shows the assessment of MAP infection status in 154 Holstein Friesian cows included in the study. Pathological examination of intestinal tissue sections allowed the classification of the animals in four groups: focal (n=55, 59.14%), multifocal (n=17, 18.28%), diffuse (n=15, 16.12%) and no lesions (n=6, 6.45%).

**Table 2**

| DIAGNOSTIC METHOD | FOCAL (n=55) | MULTIFOCAL (n=17) | DIFFUSE (n=15) | NL (n=6) | CONTROL (n=61) |
|---|---|---|---|---|---|
| ZN | 49,09% | 94.11% | 100% | 0% | UN |
| IDEXX ELISA | 5.45% | 23.52% | 73.33% | 0% | 0% |
| FECAL PCR | 9.09% | 29.41% | 73.33% | 0% | 0% |
| FECAL CULTURE | 5.45% | 17.64% | 20.00% | 0% | 0% |
| TISSUE PCR | 27.27% | 41.17% | 73.33% | 0% | UN |
| TISSUE CULTURE | 27.27% | 41.17% | 66.66% | 0% | UN |
| CLINICAL SIGNS | 0.00% | 15.38% | 64.28% | 0% | 0.00% |
| AGE (years) | 5.72±2.16 | 5.50±1.85 | 4.98±1.58 | 2.41±1.11 | 3.62±2.41 |

| | | | | | |
|---|---|---|---|---|---|
| *NL, no lesions; CONTROL; refers to the 61 control animals from a PTB-free farm; UN, undetermined (tissues not available as animals are alive); Age is expressed as the mean value of the group* ± *standard deviation. The calculation of the % of animals with clinical signs is based on animals with known clinical status (focal n=28, multifocal n=13, diffuse n=14 and control n=61).* | | | | | |

In the group of animals with focal lesions, 76.36% were positive by one or more diagnostic methods (Ziehl-Neelsen, fecal and tissue real-time PCR, fecal and tissue bacteriological culture and serum ELISA) and the remaining 23.64% were negative by all the tests assayed (n=13). Specifically, 49.09% were positive by Ziehl-Neelsen, 9.09% by fecal real-time PCR, 27.27% by tissue real-time PCR and by tissue bacteriological culture, and 5.45% by fecal bacteriological culture, and serum ELISA. None of the animals in the focal group showed clinical signs associated with PTB, so these animals with focal lesions were considered to be sub-clinically infected.

In the group of animals with multifocal lesions, 100% of the samples were positive for at least one of the following 6 techniques: Ziehl-Neelsen, fecal and tissue real-time PCR, fecal and tissue bacteriological culture and serum ELISA. In particular, 94.11% of the animals were positive by Ziehl-Neelsen, 23.52% by serum ELISA, 29.41% by fecal real-time PCR, 41.17% by tissue real-time PCR, 17.64% by fecal culture and 41.17% by tissue culture. In this group a higher percentage of animals (15.38%) showed clinical signs, including the youngest animal with clinical signs (2.72 years old). In the diffuse group, 100% of the animals were positive by Ziehl-Neelsen, 73.33% were positive by serum ELISA and by fecal and tissue real-time PCR, 20% by fecal culture and 66.66% by tissue culture. In this group, 64.28% of the animals had PTB-associated clinical signs.

Only 6 out of the 94 animals analyzed by histopathology did not show histological lesions in their intestinal tissues. These animals were negative by fecal and tissue MAP-specific PCR and bacteriological culture, and by serum ELISA. Given the difficulty to find animals without lesions it was decided to use as control group 61 animals coming from a PTB-free farm in Asturias in order to increase the size of the sample and the statistical significance of the results. The PTB-free status of this control farm had been verified yearly four times by IDEXX serum ELISA and absence of clinical cases in the period 2016-2019, as well as by bacteriological culture and specific real-time PCR of feces in 2019. However, none of them had been examined histologically since this is a post-mortem test and these animals are still alive.

Significant differences were found between the age of the focal (5.72±2.16 years) and the PTB-free control (3.62±2.41 years) group (p<0.001); and between the age of the multifocal (5.50±1.85 years) and PTB-free control (3.62±2.41 years) group (p=0.009). However, no significant differences were found between the age of the diffuse (4.98±1.58 years) and control (3.62±2.41) groups (p=0.141), focal and diffuse groups (p=0.65), multifocal and diffuse groups (p=0.905), and multifocal and focal groups (0.982).

### Example 2.2 ROC analysis of the selected biomarker-based ELISAs

The diagnostic accuracies of each biomarker-based ELISA to discriminate between the different histological groups and the control group were calculated by ROC analysis (Figures 1 and 2). ROC analysis of the data obtained from 4 biomarker-based ELISAs for detection of FAM84A, DES, MMP8 and SPACR was performed using 87 serum samples from 55 animals with focal, 17 with multifocal and 15 with diffuse lesions. ROC analysis of ABCA13-based ELISA was performed using 83 serum samples from 53 animals with focal, 16 with multifocal and 14 with diffuse lesions. As mentioned above, the control group used for this analysis consisted of 61 animals from a PTB-free farm in Asturias. The diagnostic performance of the biomarker-based ELISAs was also compared to that of conventional PTB diagnosis methods such as specific anti-MAP antibody ELISA (IDEXX ELISA). **Figure 1** shows the concentration of each biomarker in the serum of every single Holstein Friesian cattle classified within a specific histological group and **Figure 2** shows the ROC curves of the data obtained with each of the biomarker-based ELISA for each histological group.

### Example 2.2.1. Detection of animals with focal histological lesions (Table 3)

Three ELISAs for the detection of the biomarkers ABCA13, MMP8 and SPARC had good discriminatory power between the focal and control groups (0.8≤AUC<0.9). However, the ABCA13-based ELISA showed the most accurate diagnostic performance (AUC value of 0.866, p<0.001), with a sensitivity of 79.25% and a high specificity of 93.44%. Combination of ABCA13 and SPARC-based ELISAs gave an AUC value slightly higher 0.951, with a sensitivity of 93.44% and a specificity (80.77%). Diagnostic performance of ABCA13-based ELISA for the detection of animals with focal lesions was better than that of the IDEXX ELISA (AUC value of 0.536), which had a sensitivity of 14.50% and a specificity of 100.00%. No significant differences were observed between the ROC curves of the ABCA13, MMP8 and SPARC-based ELISAs (ABCA13 vs MMP8, p=0.386; ABCA13 vs. SPARC, p=0.372 and MMP8 vs. SPARC, p= 0.781). **Table 3** shows the diagnostic performance of selected biomarker-based ELISAs for diagnosis of cattle with focal histological lesions in the intestinal tissues. The ELISA with the best diagnostic performance is shown in bold face.

**Table 3**

| **FOCAL (N=55) VS. CONTROL (N=61)** | | | | | | |
|---|---|---|---|---|---|---|
| **BIOMARKER** | **AUC** | **p-value** | **CUT OFF** | **SE (%)** | **SP (%)** | **DV** |
| FAM84A | 0.522 | 0.682 | 1.04 | 38.2 | 73.8 | 0.560 |
| DES | 0.622 | 0.024 | 23.1 | 61.8 | 68.8 | 0.653 |
| **ABCA13** | **0.866** | **<0.001** | **1.74** | **79.2** | **93.4** | **0.863** |
| MMP8 | 0.802 | <0.001 | 33.31 | 87.3 | 68.8 | 0.780 |
| SPARC | 0.816 | <0.001 | 273.15 | 59.2 | 88.5 | 0.738 |
| IDEXX ELISA^{a} | 0.536 | 0.511 | 28.39 | 14.5 | 100 | 0.572 |
| IDEXX ELISA^{b} | | | 55% | 5.45 | 100 | 0.527 |

| | | | | | | |
|---|---|---|---|---|---|---|
| *AUC, area under the curve; SE, sensitivity; SP, specificity; DV, diagnostic value (semi-sum of the sensitivity and specificity); p-value, it is the p-value of the AUC area, indicates whether the discrimination between animals with focal lesions and controls is significant; DES, bovine desmin; FAM84A, bovine family with sequence similarity 84 member A; ABCA13, bovine ATP binding cassette subfamily A member 13; MMP8, bovine matrix metallopeptidase 8; SPARC, bovine secreted protein acidic and cystein rich; a, the diagnostic accuracy of the IDEXX ELISA was calculated by ROC curve analysis; b, the cut off value used to calculate the sensitivity and specificity of the assay was calculated using the cut off value established by the supplier.* | | | | | | |

### Example 2.2.2. Detection of animals with multifocal histological lesions (Table 4)

In the multifocal group the SPARC-based ELISA showed the most accurate diagnostic performance. The SPARC-based ELISA had good discriminatory power (AUC value of 0.865, p<0.001) between the multifocal and control groups, with a sensitivity of 66.70% and a specificity of 95.10%. Combination of the results using the ABCA13 and SPARC-based ELISAs gave an AUC of 0.900 with a sensitivity of 98.36% and a specificity of 64.71%. The diagnostic performance of the ELISA based on detection of SPARC was also compared to that of the IDEXX ELISA. Diagnostic performance of SPARC-based ELISA for detection of animals with multifocal lesions was better than that of the IDEXX ELISA (AUC value of 0.594), which has a sensitivity of 27.80% and a specificity of 100.00%. There were no significant differences between their ROC curves of the MMP8 and SPARC-based ELISAs (MMP8 vs. SPARC, p= 0.771).

**Table 4** shows the diagnostic performance of selected biomarkers-based ELISAs for diagnosis of cattle with multifocal histological lesions in their intestinal tissues. The ELISA with the best diagnostic performance is shown in bold face.

**Table 4**

| **MULTIFOCAL (N=17) VS. CONTROL (N=61)** | | | | | | |
|---|---|---|---|---|---|---|
| **BIOMARKER** | **AUC** | **p-value** | **CUT OFF** | **SE (%)** | **SP (%)** | **DV** |
| FAM84A | 0.524 | 0.766 | 0.87 | 50.00 | 67.20 | 0.586 |
| DES | 0.597 | 0.213 | 23.93 | 61.10 | 68.80 | 0.649 |
| ABCA13 | 0.676 | 0.028 | 0.97 | 70.60 | 70.50 | 0.705 |
| MMP8 | 0.779 | <0.001 | 26.92 | 94.40 | 62.30 | 0.783 |
| **SPARC** | **0.865** | **<0.001** | **354.94** | **66.70** | **95.10** | **0.809** |
| IDEXX ELISA^{a} | 0.594 | 0.229 | 67.58 | 27.80 | 100.00 | 0.639 |
| IDEXX ELISA^{b} | | | 55% | 27.77 | 100.00 | 0.617 |

| | | | | | | |
|---|---|---|---|---|---|---|
| *AUC, area under the curve; SE, sensitivity; SP, specificity; DV, diagnostic value (semi-sum of the sensitivity and specificity); p-value, it is the p-value of the AUC area, indicates whether the discrimination between animals with multifocal lesions and controls is significant; DES, bovine desmin; FAM84A, bovine family with sequence similarity 84 member A; ABCA13, bovine ATP binding cassette subfamily A member 13; MMP8, bovine matrix metallopeptidase 8; SPARC, bovine secreted protein acidic and cysteine rich; a, the diagnostic accuracy of the IDEXX ELISA was calculated by ROC curve analysis; b, the cut off value used to calculate the sensitivity and specificity of the assay was calculated using the cut off value established by the supplier.* | | | | | | |

### Example 2.2.3. Detection of animals with diffuse histological lesions (Table 5)

Two ELISAs for the detection of ABCA13 and MMP8 had good discriminatory power between the diffuse and control groups (0.8≤AUC<0.9). However, MMP8-based ELISA showed the most accurate diagnostic performance (AUC value of 0.866, <0.001) with a sensitivity of 100.00% and a specificity of 77.10%. In this case, combination of biomarkers-based ELISAs does not improve the results obtained individually by the MMP8-based ELISA. The diagnostic performance of the ELISA based on detection of MMP8 was also compared to that of the IDEXX ELISA. The discriminatory power of the IDEXX ELISA between the diffuse and control groups was excellent with an AUC value of 0.917 (p<0.001). The diagnostic performance of the IDEXX ELISA for the detection of animals with diffuse lesions was better than that of the biomarker-based ELISAs, with a sensitivity of 86.70% and a specificity of 96.70%. Comparison of the ROC curves of ABCA13, MMP8 and IDEXX ELISAs showed that there were no significant differences between the three ROC curves (ABCA13 vs MMP8, p=0.843; ABCA13 vs. IDEXX ELISA, p=0.482 and MMP8 vs. IDEXX ELISA, p= 0.444).

**Table 5** shows the diagnostic performance of selected biomarkers-based ELISAs for diagnosis of cattle with diffuse histological lesions in their intestinal tissues. The ELISA with the best diagnostic performance is shown in bold face.

**Table 5**

| **DIFFUSE (N=15) VS. CONTROL (N=61)** | | | | | | |
|---|---|---|---|---|---|---|
| **BIOMARKER** | **AUC** | **p-value** | **CUT OFF** | **SE (%)** | **SP (%)** | **DV** |
| FAM84A | 0.635 | 0.108 | 1.06 | 66.70 | 75.40 | 0.710 |
| DES | 0.619 | 0.159 | 36.59 | 46.70 | 88.50 | 0.676 |
| ABCA13 | 0.851 | <0.001 | 1.20 | 86.70 | 83.30 | 0.850 |
| MMP8 | 0.866 | <0.001 | 40.23 | 100.00 | 77.10 | 0.885 |
| SPARC | 0.61 | 0.192 | 7.73 | 100.00 | 36.10 | 0.680 |
| **IDEXX ELISA^{a}** | **0.917** | **<0.001** | **19.6** | **86.70** | **96.70** | **0.917** |
| IDEXX ELISA^{b} | | | 55% | 73.33 | 100 | 0.866 |

| | | | | | | |
|---|---|---|---|---|---|---|
| *AUC, area under the curve; SE, sensitivity; SP, specificity; DV, diagnostic value (semi-sum of the sensitivity and specificity); p-value, it is the p-value of the AUC area, indicates whether the discrimination between animals with diffuse lesions and controls is significant; DES, bovine desmin; FAM84A. bovine family with sequence similarity 84 member A; ABCA13, bovine ATP binding cassette subfamily A member 13; MMP8, bovine matrix metallopeptidase 8; SPARC, bovine secreted protein acidic and cysteine rich; a. the diagnostic accuracy of the IDEXX ELISA was calculated by ROC curve analysis; b. the cut off value used to calculate the sensitivity and specificity of the assay was calculated using the cut off value established by the supplier of the kit.* | | | | | | |

### Example. 2.2.4. Overall detection of animals with any type of histological lesions (Table 6)

We compared the ability of the biomarker-based ELISAs to discriminate infected cows with any type of histological lesion (focal, multifocal or diffuse) from the control group. It must be taken into account that the three different histological groups are not equally represented (focal n=55, multifocal n=17 and diffuse n=15), however, this is a reflection of the real situation in the farms. The ABCA13 and MMP8-based ELISAs had good discriminatory power (0.8≤AUC<0.9) between the animals with lesions and the control group. However, ABCA13-based ELISA showed the most accurate diagnostic performance (AUC value of 0.825, p<0.001) with a sensitivity of 70.60% and a specificity of 91.80%. The combination of the results obtained with the ABCA13 and SPARC -based ELISAs gave an AUC value slightly higher (0.933) with a sensitivity of 88.52% and a specificity of 79.76%. The diagnostic performance of the ELISA based on detection of ABCA13 was also compared to that of the IDEXX ELISA. Diagnostic performance of ABCA13-based ELISA for detection of infected animals was better than that of the IDEXX ELISA (AUC value of 0.613), which had a sensitivity of 28.40% and a specificity of 100.00%.

**Table 6** shows the diagnostic performance of selected biomarkers-based ELISAs for diagnosis of cattle with histological lesions in their intestinal tissues. The ELISA with the best diagnostic performance is shown in bold face.

**Table 6**

| **ALL LESIONS (N=93) VS. CONTROL (N=61)** | | | | | | |
|---|---|---|---|---|---|---|
| **BIOMARKER** | **AUC** | **p-value** | **CUT OFF** | **SE (%)** | **SP (%)** | **DV** |
| FAM84A | 0.542 | 0.388 | 1.06 | 42.00 | 75.40 | 0.587 |
| DES | 0.617 | 0.016 | 23.1 | 61.40 | 68.80 | 0.651 |
| **ABCA13** | **0.825** | **<0.001** | **1.29** | **70.60** | **91.80** | **0.812** |
| MMP8 | 0.808 | <0.001 | 33.17 | 87.50 | 68.80 | 0.782 |
| SPARC | 0.79 | <0.001 | 354.94 | 47.10 | 95.10 | 0.711 |
| IDEXX ELISA^{a} | 0.613 | 0.020 | 28.39 | 28.40 | 100.00 | 0.642 |
| IDEXX ELISA^{b} | | | 55% | 32.20 | 100.00 | 0.661 |

| | | | | | | |
|---|---|---|---|---|---|---|
| *AUC, area under the curve; SE, sensitivity; SP, specificity; DV, diagnostic value (semi-sum of the sensitivity and specificity); p-value, it is the p-value of the AUC area, indicates whether the discrimination between animals with lesions and controls is significant; DES, bovine desmin; FAM84A. bovine family with sequence similarity 84 member A; ABCA13, bovine ATP binding cassette subfamily A member 13; MMP8, bovine matrix metallopeptidase 8; SPARC, bovine secreted protein acidic and cysteine rich; a, the diagnostic accuracy of the IDEXX ELISA was calculated by ROC curve analysis; b, the cut off value used to calculate the sensitivity and specificity of the assay was calculated using the cut off value established by the supplier.* | | | | | | |

A summary of the ELISAs with the best diagnostic performance for each histological group is provided below. ABCA13-based ELISA showed the most accurate diagnostic performance (AUC value of 0.866,p<0.001) for detection of animals with focal lesions. SPARC-based ELISA showed the most accurate diagnostic performance (AUC value of 0.865, p<0.001) for the detection of animals with multifocal lesions. MMP8-based ELISA showed the most accurate diagnostic performance (AUC value of 0.866, p<0.001) for the detection of animals with diffuse lesions, however, the diagnostic performance of the IDEXX ELISA (AUC value of 0.917, p<0.001) was better than that of the biomarker-based ELISAs with a sensitivity of 86.70% and a specificity of 96.70%. Finally, for overall detection of animals with any type of histological lesions ABCA13-based ELISA showed the most accurate diagnostic performance (AUC value of 0.825, p<0.001) with a sensitivity of 70.60% and a specificity of 91.80%. Moreover, the combination of biomarkers for detection of animals with focal, multifocal or any type of lesions could improve the sensitivity of the diagnosis. Taken together our results show that the ABCA13-based ELISA is a very accurate method for the discrimination of animals with focal lesions and for overall detection of animals with any type of histological lesion.

**Table 7** shows the diagnostic performance of the best biomarker-based ELISAs and IDEXX ELISA for the detection of the different histological animal groups. ROC analysis of MMP8 and SPARC-based ELISAs and the IDEXX ELISA was performed using 87 serum samples from 55 animals with focal, 17 with multifocal and 15 with diffuse lesions. ROC analysis of ABCA13-based ELISA was performed using 83 serum samples from 53 animals with focal, 16 with multifocal and 14 with diffuse lesions:

**Table 7**

| **GROUP** | **ELISA** | **AUC** | **p-value** | **CUT OFF** | **SE (%)** | **SP (%)** | **DV** |
|---|---|---|---|---|---|---|---|
| FOCAL | ABCA13 | 0.866 | <0.001 | 1.74 | 79.20 | 93.40 | 0.863 |
| MULTIFOCAL | SPARC | 0.676 | 0.028 | 0.97 | 70.60 | 70.50 | 0.705 |
| DIFFUSE | MMP8 | 0.851 | <0.001 | 1.20 | 86.70 | 83.30 | 0.850 |
| DIFFUSE | IDEXX^{a} | 0.917 | <0.001 | 19.6 | 86.70 | 96.70 | 0.917 |
| ALL LESIONS | ABCA13 | 0.825 | <0.001 | 1.29 | 70.60 | 91.80 | 0.812 |

| | | | | | | | |
|---|---|---|---|---|---|---|---|
| *GROUP, refers to the different groups of analysis established based on histopathological classification; AUC, area under the curve; p-value, indicates whether the discrimination between animals with lesions and controls is significant; SE, sensitivity; SP, specificity; DV, diagnostic value (semi-sum of the sensitivity and specificity); ABCA13, bovine ATP binding cassette subfamily A member 13; MMP8, bovine matrix metallopeptidase 8; SPARC, bovine secreted protein acidic and cysteine rich; a, the diagnostic accuracy of the IDEXX ELISA was calculated by ROC curve analysis* | | | | | | | |

### Example 2.2.5. Comparison of the diagnostic performance of the ABCA13-based ELISA with that of other conventional methods for the diagnostic of animals with focal lesions

The diagnostic performance of the ELISA based on detection of ABCA13 was also compared to that of conventional PTB diagnosis methods such as specific anti-MAP antibody ELISA (IDEXX ELISA), and specific fecal and tissue real-time PCR and bacteriological culture.

**Table 8** shows the diagnostic performance of conventional and novel biomarker-based diagnostic assays for the detection of animals with focal lesions in their intestinal tissues. ABCA13-based ELISA showed a better diagnostic value than the other diagnostic methods tested for the detection and discrimination of animals with focal lesions. It was able to detect as positive 44 out of 55 animals with focal lesions (80% sensitivity) and as negative 57 out of the 61 controls (93% specificity). The diagnostic value or sensitivities of the other conventional methodologies was lower. Specifically, the IDEXX ELISA was 100% specific but only detected as positive 3 out of 55 animals (5.45% sensitivity) when the cut off used (55%) was the one established by the supplier or 8 out of 55 (14.54%) when we used the cut-off point (28.39%) calculated by ROC analysis.

**Table 8**

| **METHOD** | **AUC** | **p-value** | **CUT OFF** | **SE (%)** | **SP (%)^{d}** | **DV** |
|---|---|---|---|---|---|---|
| ELISA ABCA13^{a} | 0.866 | <0.001 | 1.74 | 79.20 | 93.40 | 0.863 |
| IDEXX ELISA^{b} | 0.536 | 0.511 | 28.39% | 14.50 | 100 | 0.572 |
| IDEXX ELISA^{c} | | | 55% | 5.45 | 100 | 0.527 |
| Fecal culture | | | | 5.45 | 100 | 0.527 |
| Fecal PCR | | | | 9.09 | 100 | 0.545 |
| Tissue culture | | | | 27.27 | UN | |
| Tissue PCR | | | | 27.27 | UN | |

| | | | | | | |
|---|---|---|---|---|---|---|
| *AUC, area under the curve; SE, sensitivity; SP, specificity; DV, diagnostic value; UN, undetermined; p-value, indicates whether the discrimination between animals with focal lesions and controls is significant. ABCA13, bovine ATP binding cassette subfamily A member 13; a, indicate that the number of animals with focal, multifocal, diffuse or with any type of lesions analysed with theABCA13-based ELISA was 53, 16, 14 and 83, respectively; b, the cut off value used to estimate the sensitivity and specificity of the assay was calculated by ROC analysis; c, the cut off value used to estimate the sensitivity and specificity of the assay was the one established by the supplier; d, the control group used to calculate the specificity consisted of 61 animals from a PTB-free farm.* | | | | | | |

### Example 2.2.6. A combination of biomarker-based ELISAs was tested to determine the diagnostic value of the ABCA13-based ELISA using Logistic regression analysis

Diagnostic models based on the combination of some of the five selected biomarkers slightly improve the diagnostic value of the single biomarker-based ELISAs (see **Table 9**).

**Table 9** shows the diagnostic performance of models based on the combined use of various biomarkers compared with the performance obtained for single biomarker-based ELISAs for diagnosis of cattle with different histological lesions in the intestinal tissues. This analysis was carried out using 55 focal, 18 multifocal, 15 diffuse animals and 67 control animals.

**Table 9**

| **GROUP** | **BIOMARKER** | **AUC** | **P-value** | **SE (%)** | **SP (%)** | **DV** |
|---|---|---|---|---|---|---|
| FOCAL | ABCA13 | 0.837 | <0.001 | 79.25 | 88.06 | 0.84 |
| MULTIFOCAL | SPARC | 0.852 | <0.001 | 66.67 | 92.54 | 0.80 |
| DIFFUSE | MMP8 | 0.831 | <0.001 | 100 | 71.64 | 0.86 |
| PATENT | MMP9 | 0.781 | <0.001 | 96.97 | 58.21 | 0.78 |
| ANY LESION | ABCA13 | 0.793 | <0.001 | 69.41 | 86.57 | 0.78 |

| **GROUP** | **BIOMARKER MODELS** | **AUC** | **95%CI** | **SE (%)** | **SP (%)** | **DV** |
|---|---|---|---|---|---|---|
| FOCAL | DES, ABCA13 AND SPARC | 0.911 | 0.437-0.646 | 84.62 | 86.57 | 0.86 |
| MULTIFOCAL | DES, FAM84A AND SPARC | 0.851 | 0.737-0.964 | 70.54 | 89.55 | 0.80 |
| DIFFUSE | - | | | | | |
| PATENT | DES, ABCA13, MMP8 AND SPARC | 0.82 | 0.727-0.910 | 75 | 85.07 | 0.80 |
| ANY LESION | DES, ABCA13, MMP8 AND SPARC | 0.878 | 0.824-0.932 | 80.95 | 85.07 | 0.83 |

| | | | | | | |
|---|---|---|---|---|---|---|
| *Group, histological group; AUC, area under the curve; SE, sensitivity; SP, specificity; DV, diagnostic value (semi-sum of the sensitivity and specificity); p-value, it is the p-value of the AUC area, indicates whether the discrimination between animals with focal lesions and Controls is significant; DES, bovine desmin; FAM84A, bovine family with sequence similarity 84 member A; ABCA13, bovine ATP binding cassette subfamily A member 13; MMP8, bovine matrix metallopeptidase 8; SPARC, bovine secreted protein acidic and cystein rich.* | | | | | | |

### Example 2.2.7. Large-scale validation of ABCA13-based ELISA

We had shown that the ELISA for the detection of ABCA13 is an accurate method for detection of animals with focal lesions and for overall detection of animals with any type of histological lesion.

To further confirm these results a large-scale validation of the ABC13-based ELISA (n=661) was performed using 556 serum samples from 445 infected animals with focal, 55 with multifocal and 58 with diffuse lesions. The non-infected control group consisted of 105 animals from two PTB-free farms in Asturias.

**Table 10** shows the diagnostic performance of the ABCA13-based ELISA for diagnosis of cattle with different types of histological lesions in their intestinal tissues. The diagnostic accuracy of the ABCA13-based ELISA was calculated by ROC curve analysis.

**Table 10**

| | **AUC** | **P value** | **CUTOFF** | **SE %** | **SP%** | **DV** |
|---|---|---|---|---|---|---|
| Control (n=105) vs. All-lesion (n=556) | 0.843 | <0.0001 | >3.66 | 77.24 | 84.76 | 0.81 |
| Control (n=105) vs. focal (n=445) | 0.883 | <0.0001 | >3.66 | 82.02 | 84.76 | 0.83 |
| Control (n=105) vs. multifocal (n=55) | 0.667 | 0.0032 | >3.66 | 61.82 | 84.76 | 0.73 |
| Control (n=105) vs. diffuse (n=58) | 0.705 | <0.0001 | >3.1 | 63,79 | 77.10 | 0.70 |
| Control (n=105) vs. Patent (n=113) | 0.687 | <0.0001 | >3.66 | 58.41 | 84.76 | 0.72 |

| | | | | | | |
|---|---|---|---|---|---|---|
| *AUC, area under the curve; SE, sensitivity; SP, specificity; DV, diagnostic value (semi-sum of the sensitivity and specificity); p-value, it is the p-value of the AUC area, indicates whether the discrimination between animals with focal lesions and Controls is significant; ABCA13, bovine ATP binding cassette subfamily A member 13. The patent group consisted of the animals with multifocal and diffuse lesions; The discriminatory power of each biomarker to discern between the different histopathological groups and the Control group was considered as follows: biomarker-based ELISAs with AUC values ≥0.9 were considered to have excellent discriminatory power; 0.8≤AUC<0.9 good discriminatory power; 0.7≤AUC <0.8fair discriminatory power; and AUC <0.7, poor discriminatory.* | | | | | | |

The ELISA for detection of ABCA13 has a good discriminatory power between the focal and the control group (AUC value of 0.843, p<0.0001) and between the all-lesion group and the control (AUC value of 0.883, p<0.0001). These results further confirm that the ELISA for the detection of ABCA13 is an accurate diagnostic method for detection of animals with focal lesions and for overall detection of animals with any type of histological lesion.

### Example 2.2.8. Comparison between the results obtained with ABCA13-based ELISA and IDEXX ELISA (N=661)

As we have mentioned the IDEXX ELISA is nowadays widely used for the diagnosis of PTB although this method is associated with a high rate of false negative results (low sensitivity). For this reason, we have compared the results obtained with the ABCA13-based ELISA and the IDEXX ELISA for the 661 samples used in the large-scale validation study.

**Table** 11 shows the sensitivity, specificity, and diagnostic value of the novel ABCA13-based ELISA and the conventional IDEXX ELISA for the detection of the different histological groups. The cut-off value used to estimate the sensitivity and the specificity of the ABCA13-based ELISA and IDEXX ELISA were 3,66 ng/mL and 55% (relative % ODsample/ODpositive control), respectively. The estimation of the sensitivity was based on the analysis of 445 animals with focal lesions, 55 with multifocal, and 58 with diffuse. The control group used to calculate the specificity consisted of 105 animals from two PTB-free farms.

**Table 11**

| | **Sensitivity (%)** | |
|---|---|---|
| LESION TYPE | ABCA13-based ELISA | IDEX ELISA |
| FOCAL | 82,02 | 3,37 |
| MULTIFOCAL | 61,82 | 32,72 |
| DIFFUSE | 63,8 | 86,2 |
| MULTIFOCAL+DIFFUSE | 58,41 | 60,17 |
| ALL LESION TYPES | 77,24 | 15,09 |

| | **Specificity (%)** | |
|---|---|---|
| | ABCA13-based ELISA | IDEXX ELISA |
| FOCAL | 84,76 | 100 |
| MULTIFOCAL | 84,76 | 100 |
| DIFFUSE | 77,1 | 100 |
| MULTIFOCAL+DIFFUSE | 84,76 | 100 |
| ALL LESION TYPES | 84,76 | 100 |

| | **Diagnostic value** | |
|---|---|---|
| | ABCA13-based ELISA | IDEX ELISA |
| FOCAL | 0,83 | 0,52 |
| MULTIFOCAL | 0,73 | 0,66 |
| DIFFUSE | 0,70 | 0,93 |
| MULTIFOCAL+DIFFUSE | 0,72 | 0,80 |
| ALL LESION TYPES | 0,81 | 0,58 |

| | | |
|---|---|---|
| *ABCA13, bovine ATP binding cassette subfamily A member 13* | | |

These findings support our previous results indicating that the ABCA13-based ELISA is a very accurate method for the discrimination of animals with focal lesions and for overall detection of animals with any type of histological lesion. Specifically, the ABC13-based ELISA has a higher sensitivity for detection of animals with focal lesions representing subclinical infections difficult to detect by conventional diagnostic methods.

## Claims

1. *In vitro* method for diagnosing subjects infected with *Mycobacterium* species which comprises:
a) Measuring the concentration of the protein ABCA13 in a biological sample consisting of serum or plasma obtained from the subject, and
b) Wherein if an increase of the concentration of the protein ABCA13 is identified in the step a) with respect to the concentration of the protein ABCA13 measured in uninfected control subjects, it is an indication that the subject is infected with *Mycobacterium* species.

2. *In vitro* method, according to claim 1, for diagnosing subjects infected with *Mycobacterium avium subsp. paratuberculosis* which comprises:
a) Measuring the concentration of the protein ABCA13 in a biological sample consisting of serum or plasma obtained from the subject, and
b) Wherein if an increase of the concentration of the protein ABCA13 is identified in the step a) with respect to the concentration of the protein ABCA13 measured in uninfected control subjects, it is an indication that the subject is infected with *Mycobacterium avium subsp. paratuberculosis.*

3. *In vitro* method, according to any of the previous claims, for the diagnosis of paratuberculosis in a subject which comprises:
a) Measuring the concentration of the protein ABCA13 in a biological sample consisting of serum or plasma obtained from the subject, and
b) Wherein if an increase of the concentration of the protein ABCA13 is identified in the step a) with respect to the concentration of the protein ABCA13 measured in uninfected control subjects, it is an indication that the subject is suffering from paratuberculosis.

4. *In vitro* method, according to any of the previous claims, which comprises:
a) Measuring the concentration of the proteins [ABCA13 and SPARC] in a biological sample consisting of serum or plasma obtained from the subject, and
b) Wherein if an increase of the concentration of the proteins [ABCA13 and SPARC] is identified in the step a) with respect to the concentration of the proteins [ABCA13 and SPARC] measured in uninfected control subjects, it is an indication that the subject is suffering from paratuberculosis.

5. *In vitro* method, according to any of the previous claims, which comprises:
a) Measuring the concentration of the proteins [ABCA13 and SPARC and MMP8] in a biological sample consisting of serum or plasma obtained from the subject, and
b) Wherein if an increase of the concentration of the proteins [ABCA13 and SPARC and MMP8] is identified in the step a) with respect to the concentration of the proteins [ABCA13 and SPARC and MMP8] measured in uninfected control subjects, it is an indication that the subject is suffering from paratuberculosis.

6. *In vitro* method, according to any of the previous claims, which comprises:
a) Measuring the concentration of the proteins of any of the claims 1 to 5, in a biological sample consisting of serum or plasma obtained from the subject,
b) Wherein if the concentration obtained for any of the previously cited proteins is above the established cut-off value, this is indicative that the subject is suffering from paratuberculosis.

7. *In vitro* method, according to any of the previous claims, for the diagnosis of latent or patent paratuberculosis, preferably latent paratuberculosis.

8. *In vitro* method, according to any of the previous claims, wherein the subject is a mammal selected from the group consisting of: cattle, goat, sheep, horse, pig, deer, rabbit, wild boar, bison, llama, alpaca, opossum, badger, elephant or human being; preferably a farm-animal selected from the group consisting of: cow, goat, sheep, horse, pig, deer, llama or alpaca.

9. *In vitro* use of the protein ABCA13, of the proteins [ABCA13 and SPARC], or of the proteins [ABCA13 and SPARC and MMP8], in a biological sample consisting of serum or plasma, for diagnosing subjects infected with *Mycobacterium* species.

10. *In vitro* use, according to claim 9, for diagnosing subjects infected with *Mycobacterium avium subsp.paratuberculosis.*

11. *In vitro* use, according to any of the claims 9 or 10, for the diagnosis of paratuberculosis.

12. *In vitro* use, according to any of the claims 9 to 11, for the diagnosis of latent or patent paratuberculosis, preferably latent paratuberculosis.

13. Use of a kit which comprises tools or reagents for determining the concentration of the protein ABCA13 in a biological sample consisting of serum or plasma for diagnosing subjects infected with *Mycobacterium* species.

14. Use of a kit, according to claim 13, which comprises tools or reagents for determining the concentration of the proteins [ABCA13 and SPARC] or the proteins [ABCA13 and SPARC and MMP8] for diagnosing subjects infected with *Mycobacterium* species.

## Patentansprüche

1. In-vitro-Verfahren zur Diagnose von mit *Mycobacterium-Spezies* infizierten Individuen, welches Folgendes umfasst:
a) das Messen der Konzentration des Proteins ABCA13 in einer biologischen Probe bestehend aus Serum oder Plasma erhalten aus dem Individuum, und
b) wobei, wenn eine Erhöhung der Konzentration des Proteins ABCA13 in Schritt a) identifiziert wird, in Bezug auf die Konzentration des Proteins ABCA13 gemessen in nichtinfizierten Kontrollindividuen, dies ein Hinweis ist, dass das Individuum mit *Mycobacterium-Spezies* infiziert ist.

2. In-vitro-Verfahren nach Anspruch 1, zur Diagnose von mit *Mycobacterium avium subsp. paratuberculosis* infizierten Individuen, welches Folgendes umfasst:
a) das Messen der Konzentration des Proteins ABCA13 in einer biologischen Probe bestehend aus Serum oder Plasma erhalten aus dem Individuum, und
b) wobei, wenn eine Erhöhung der Konzentration des Proteins ABCA13 in Schritt a) identifiziert wird, in Bezug auf die Konzentration des Proteins ABCA13 gemessen in nichtinfizierten Kontrollindividuen, dies ein Hinweis ist, dass das Individuum mit *Mycobacterium avium subsp. paratuberculosis* infiziert ist.

3. In-vitro-Verfahren nach einem der vorhergehenden Ansprüche, zur Diagnose von Paratuberkulose in einem Individuum, welches Folgendes umfasst:
a) das Messen der Konzentration des Proteins ABCA13 in einer biologischen Probe bestehend aus Serum oder Plasma erhalten aus dem Individuum, und
b) wobei, wenn eine Erhöhung der Konzentration des Proteins ABCA13 in Schritt a) identifiziert wird, in Bezug auf die Konzentration des Proteins ABCA13 gemessen in nichtinfizierten Kontrollindividuen, dies ein Hinweis ist, dass das Individuum unter Paratuberkulose leidet.

4. In-vitro-Verfahren nach einem der vorhergehenden Ansprüche, welches Folgendes umfasst:
a) das Messen der Konzentration der Proteine [ABCA13 und SPARC] in einer biologischen Probe bestehend aus Serum oder Plasma erhalten aus dem Individuum, und
b) wobei, wenn eine Erhöhung der Konzentration der Proteine [ABCA13 und SPARC] in Schritt a) identifiziert wird, in Bezug auf die Konzentration der Proteine [ABCA13 und SPARC] gemessen in nichtinfizierten Kontrollindividuen, dies ein Hinweis ist, dass das Individuum unter Paratuberkulose leidet.

5. In-vitro-Verfahren nach einem der vorhergehenden Ansprüche, welches Folgendes umfasst:
a) das Messen der Konzentration der Proteine [ABCA13 und SPARC und MMP8] in einer biologischen Probe bestehend aus Serum oder Plasma erhalten aus dem Individuum, und
b) wobei, wenn eine Erhöhung der Konzentration der Proteine [ABCA13 und SPARC und MMP8] in Schritt a) identifiziert wird, in Bezug auf die Konzentration der Proteine [ABCA13 und SPARC und MMP8] gemessen in nichtinfizierten Kontrollindividuen, dies ein Hinweis ist, dass das Individuum unter Paratuberkulose leidet.

6. In-vitro-Verfahren nach einem der vorhergehenden Ansprüche, welches Folgendes umfasst:
a) das Messen der Konzentration der Proteine nach einem der Ansprüche 1 bis 5, in einer biologischen Probe bestehend aus Serum oder Plasma erhalten aus dem Individuum,
b) wobei, wenn die für irgendeines der zuvor erwähnten Proteine erhaltene Konzentration über dem festgelegten Berücksichtigungsgrenzwert liegt, dies darauf schließen lässt, dass das Individuum unter Paratuberkulose leidet.

7. In-vitro-Verfahren nach einem der vorhergehenden Ansprüche, zur Diagnose von latenter oder offener Paratuberkulose, vorzugsweise latenter Paratuberkulose.

8. In-vitro-Verfahren nach einem der vorhergehenden Ansprüche, wobei das Individuum ein Säugetier ist, ausgewählt aus der Gruppe bestehend aus: Rind, Ziege, Schaf, Pferd, Schwein, Hirsch, Kaninchen, Wildschwein, Bison, Lama, Alpaka, Beutelratte, Dachs, Elefant oder Mensch; vorzugsweise ein landwirtschaftliches Nutztier ausgewählt aus der Gruppe bestehend aus: Kuh, Ziege, Schaf, Pferd, Schwein, Hirsch, Lama oder Alpaka.

9. *In-vitro*-Verwendung des Proteins ABCA13, der Proteine [ABCA13 und SPARC] oder der Proteine [ABCA13 und SPARC und MMP8], in einer biologischen Probe bestehend aus Serum oder Plasma, zur Diagnose von mit *Mycobacterium-Spezies* infizierten Individuen.

10. *In-vitro*-Verwendung nach Anspruch 9, zur Diagnose von mit *Mycobacterium avium subsp. paratuberculosis* infizierten Individuen.

11. In-vitro-Verwendung nach einem der Ansprüche 9 oder 10, zur Diagnose von Paratuberkulose.

12. *In-vitro*-Verwendung nach einem der Ansprüche 9 bis 11, zur Diagnose von latenter oder offener Paratuberkulose, vorzugsweise latenter Paratuberkulose.

13. Verwendung eines Kits, welches Werkzeuge oder Reagenzien umfasst, zum Bestimmen der Konzentration des Proteins ABCA13 in einer biologischen Probe bestehend aus Serum oder Plasma zur Diagnose von mit *Mycobacterium-Spezies* infizierten Individuen.

14. Verwendung eines Kits nach Anspruch 13, welches Werkzeuge oder Reagenzien umfasst, zum Bestimmen der Konzentration der Proteine [ABCA13 und SPARC] oder der Proteine [ABCA13 und SPARC und MMP8] zur Diagnose von mit *Mycobacterium-*Spezies infizierten Individuen.

## Revendications

1. Procédé *in vitro* pour le diagnostic des sujets infectés par des espèces de *Mycobacterium* qui comprend:
a) mesurer la concentration de la protéine ABCA13 dans un échantillon biologique consistant en sérum ou en plasma obtenu à partir du sujet, et
b) dans lequel si une augmentation de la concentration de la protéine ABCA13 est identifiée dans l'étape a) par rapport à la concentration de la protéine ABCA13 mesurée chez des sujets contrôle non infectés, c'est une indication que le sujet est infecté par des espèces de *Mycobacterium.*

2. Procédé *in vitro,* selon la revendication 1, pour le diagnostic des sujets infectés par *Mycobacterium avium subsp. paratuberculosis* qui comprend:
a) mesurer la concentration de la protéine ABCA13 dans un échantillon biologique consistant en sérum ou en plasma obtenu à partir du sujet, et
b) dans lequel si une augmentation de la concentration de la protéine ABCA13 est identifiée dans l'étape a) par rapport à la concentration de la protéine ABCA13 mesurée chez des sujets contrôle non infectés, c'est une indication que le sujet est infecté par *Mycobacterium avium subsp. paratuberculosis.*

3. Procédé *in vitro,* selon l'une quelconque des revendications précédentes, pour le diagnostic de paratuberculose chez un sujet qui comprend:
a) mesurer la concentration de la protéine ABCA13 dans un échantillon biologique consistant en sérum ou en plasma obtenu à partir du sujet, et
b) dans lequel si une augmentation de la concentration de la protéine ABCA13 est identifiée dans l'étape a) par rapport à la concentration de la protéine ABCA13 mesurée chez des sujets contrôle non infectés, c'est une indication que le sujet souffre de paratuberculose.

4. Procédé *in vitro,* selon l'une quelconque des revendications précédentes, qui comprend:
a) mesurer la concentration des protéines [ABCA13 et SPARC] dans un échantillon biologique consistant en sérum ou en plasma obtenu à partir du sujet, et
b) dans lequel si une augmentation de la concentration des protéines [ABCA13 et SPARC] est identifiée dans l'étape a) par rapport à la concentration des protéines [ABCA13 et SPARC] mesurée chez des sujets contrôle non infectés, c'est une indication que le sujet souffre de paratuberculose.

5. Procédé *in vitro,* selon l'une quelconque des revendications précédentes, qui comprend:
a) mesurer la concentration des protéines [ABCA13 et SPARC et MMP8] dans un échantillon biologique consistant en sérum ou en plasma obtenu à partir du sujet, et
b) dans lequel si une augmentation de la concentration des protéines [ABCA13 et SPARC et MMP8] est identifiée dans l'étape a) par rapport à la concentration des protéines [ABCA13 et SPARC et MMP8] mesurée chez des sujets contrôle non infectés, c'est une indication que le sujet souffre de paratuberculose.

6. Procédé *in vitro,* selon l'une quelconque des revendications précédentes, qui comprend:
a) mesurer la concentration des protéines selon l'une quelconque des revendications 1 à 5, dans un échantillon biologique consistant en sérum ou en plasma obtenu à partir du sujet,
b) dans lequel si la concentration obtenue pour l'une quelconque des protéines citées précédemment est au-dessus de la valeur seuil établie, c'est une indication que le sujet souffre de paratuberculose.

7. Procédé *in vitro,* selon l'une quelconque des revendications précédentes, pour le diagnostic de la paratuberculose latente ou patente, préférablement paratuberculose latente.

8. Procédé *in vitro,* selon l'une quelconque des revendications précédentes, dans lequel le sujet est un mammifère choisi parmi le groupe consistant en : bovin, chèvre, mouton, cheval, porc, cervidé, lapin, sanglier sauvage, bison, lama, alpaga, opossum, blaireau, éléphant ou être humain ; préférablement un animal de ferme choisi parmi le groupe consistant en : vache, chèvre, mouton, cheval, porc, cervidé, lama ou alpaga.

9. Utilisation *in vitro* de la protéine ABCA13, des protéines [ABCA13 et SPARC], ou des protéines [ABCA13 et SPARC et MMP8], dans un échantillon biologique consistant en sérum ou en plasma, pour le diagnostic des sujets infectés par des espèces de *Mycobacterium.*

10. Utilisation *in vitro,* selon la revendication 9, pour le diagnostic de sujets infectés par *Mycobacterium avium subsp. paratuberculosis.*

11. Utilisation *in vitro,* selon l'une quelconque des revendications 9 ou 10, pour le diagnostic de paratuberculose.

12. Utilisation *in vitro,* selon l'une quelconque des revendications 9 à 11, pour le diagnostic de la paratuberculose latente ou patente, préférablement paratuberculose latente.

13. Utilisation d'un kit qui comprend des outils ou réactifs pour déterminer la concentration de la protéine ABCA13 dans un échantillon biologique consistant en sérum ou en plasma pour le diagnostic de sujets infectés par des espèces de *Mycobacterium.*

14. Utilisation d'un kit, selon la revendication 13, qui comprend des outils ou réactifs pour déterminer la concentration des protéines [ABCA13 et SPARC] ou des protéines [ABCA13 et SPARC et MMP8] pour le diagnostic des sujets infectés par des espèces de *Mycobacterium.*
